# FASCICULE DE BREVET EUROPEEN

(11) **EP 1 732 453 B1**
(45) Date de publication et mention de la délivrance du brevet: **27.04.2016**
(21) Numéro de dépôt: 05757150.7
(22) Date de dépôt: 11.04.2005
(51) Int. Cl.: A61B 17/16, A61B 5/053

(54) **DISPOSITIF POUR LE SUIVI DE LA PÉNÉTRATION D'UN INSTRUMENT DANS UNE STRUCTURE ANATOMIQUE**
VORRICHTUNG ZUR VERFOLGUNG DER PENETRATION EINES INSTRUMENTS IN EINER ANATOMISCHEN STRUKTUR
DEVICE FOR FOLLOWING THE PENETRATION OF AN INSTRUMENT IN AN ANATOMICAL STRUCTURE

(30) Priorité: 09.04.2004 FR 0450721
(43) Date de publication de la demande: 20.12.2006
(73) Titulaire: SPINEGUARD, 94160 Saint Mandé (FR)
(72) Inventeur: ZELLER, Reinhard, F-92100 Boulogne-Billancourt (FR); BRAYDA-BRUNO, Marco, IT-20154 Milano (IT); PETIT, Dominique, F-62180 Verton (FR); VANACKER, Gérard, Tiburon CA 94290 (US); BOURLION, Maurice, F-42400 Saint-Chamond (FR)
(74) Mandataire: Cabinet Plasseraud
(86) Numéro de dépôt international: PCT/FR2005/000873
(87) Numéro de publication internationale: WO 2005/102183

(56) Documents cités:
- EP-A- 0 607 688
- WO-A-03/068076
- DE-A- 4 232 487
- DE-C- 19 549 662
- US-A- 4 243 388
- US-A- 5 112 224
- US-B1- 6 337 994

## Description

La présente invention concerne le domaine de la chirurgie rachidienne, et notamment les instruments pour le perçage vertébral, cervical, thoracique, lombaire sacré ou ilio sacré.

L'invention concerne plus particulièrement un dispositif pour le suivi de la pénétration d'un instrument dans une structure anatomique, en particulier une structure osseuse, comportant une source de tension alimentant au moins deux électrodes, une desdites électrodes au moins étant portée par la partie de l'instrument destinée à pénétrer dans la structure anatomique, ainsi qu'un moyen de mesure de l'impédance entre lesdites électrodes, ou la combinaison électrostimulateur moyen de mesure de l'impédance.

De tels dispositifs permettent de suivre la progression du perçage par l'analyse du signal électrique détecté, dont le niveau varie en fonction de la nature de la matière biologique en contact avec les électrodes placées sur l'instrument. Lors de la formation d'une brèche dans la matière osseuse, l'impédance décroît brutalement, ce qui permet à l'opérateur de mieux maîtriser la pénétration de l'instrument de perçage et d'éviter la lésion de la moelle épinière ou de nerfs en sortant de la trajectoire optimale.

De tels dispositifs sont utilisés notamment pour la préparation de la pose de vis de fixation osseuse. Dans ce cadre, il s'avère souvent nécessaire d'injecter dans la cavité osseuse un matériau fluide ou semi-fluide, comme par exemple du ciment osseux destiné à consolider la structure osseuse. Il doit alors être procédé à une succession d'interventions avec des ancillaires distincts, et à une répétition de gestes chirurgicaux invasifs.

Le document US 6 337 994 décrit un dispositif selon le préambule de la revendication 1.

Le but de la présente invention est de proposer un dispositif modulaire permettant de réaliser, à partir d'un seul geste chirurgical invasif, une succession de gestes chirurgicaux, comme la pose de vis cannelée.

A cet effet, l'invention concerne selon son acception la plus générale un dispositif pour le suivi de la pénétration d'un instrument dans une structure anatomique, en particulier une structure osseuse, tel que défini dans la revendication 1.

Ainsi, selon l'emplacement de la partie amovible dans la partie pénétrante, certains des gestes chirurgicaux succédant l'opération de pénétration (pose d'une vis cannelée, injection d'un ciment osseux, ...), peuvent être exécutés sans qu'il soit nécessaire de recourir à un autre geste chirurgical invasif autre que le geste de pénétration dans la structure osseuse par la partie pénétrante dudit instrument.

Plus spécifiquement, selon que la partie amovible constitue une partie interne ou la périphérie latérale de la partie pénétrante de l'instrument de pénétration, ladite partie pénétrante est adaptée pour présenter, ou bien un canal opérateur, ou bien une âme de guidage.

Ainsi, selon une configuration qui n'est pas l'invention, la partie pénétrante dudit instrument de pénétration comporte un canal opérateur recevant la partie longitudinale amovible. Le retrait de la partie longitudinale de la partie pénétrante conduit à la formation du canal opérateur.

De préférence, le canal opérateur est constitué par une électrode amovible.

Avantageusement, le dispositif comporte une broche de guidage, souple ou rigide, ou un tube d'injection de fluide traversant ledit canal opérateur.

Selon l'invention, la partie longitudinale amovible constitue la périphérie latérale de la partie pénétrante dudit instrument de pénétration. Ainsi, lorsque la partie longitudinale est retirée de la partie pénétrante, cette dernière présente une âme de guidage.

Avantageusement, l'âme de guidage consiste en une électrode.

Avantageusement, l'âme de guidage est centrale.

Avantageusement, la partie longitudinale amovible est une électrode.

L'invention sera mieux comprise à la lecture de la description qui suit, se référant aux figures annexées concernant des exemples non limitatifs de réalisation, où :
- la figure 1 représente une vue en coupe longitudinale partielle de la partie pénétrante d'un instrument de pénétration constituant un dispositif selon l'invention ;
- la figure 2 représente une vue en coupe longitudinale partielle de la partie pénétrante de l'instrument de pénétration de la figure 1 présentant un canal opérateur ; et
- la figure 3 représente une vue en coupe longitudinale partielle de la partie pénétrante de l'instrument de pénétration de la figure 1 présentant une âme de guidage.

Le dispositif selon l'invention est destiné au suivi de la pénétration d'un instrument dans une structure anatomique, et en particulier dans une structure osseuse.

Ledit dispositif comporte, outre ledit instrument de pénétration, une source de tension alimentant deux électrodes ainsi qu'un moyen de mesure de l'impédance entre lesdites électrodes.

Afin d'alerter en cas de variations d'impédance traduisant la formation de brèche dans la structure osseuse lors de l'opération de pénétration avec ledit instrument, l'une au moins desdites électrodes est portée par la partie de l'instrument de pénétration destinée à pénétrer dans la partie osseuse.

Dans les exemples de réalisation qui suivent, la partie pénétrante dudit instrument de pénétration porte avantageusement les deux électrodes offrant ainsi un dispositif autonome, ne nécessitant aucun câblage externe.

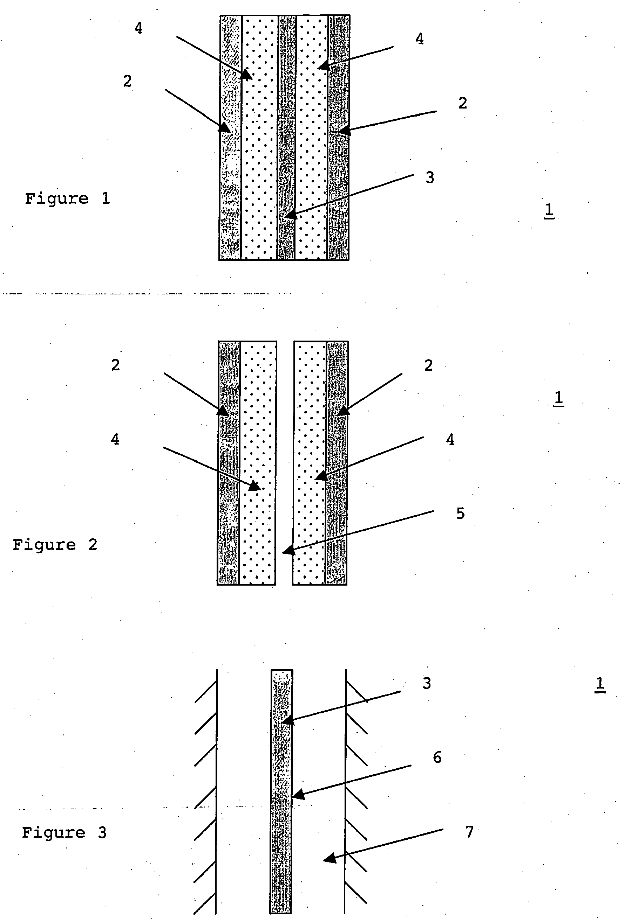

La figure 1 représente une vue en coupe longitudinale partielle de la partie pénétrante (1) dudit instrument de pénétration.

Ladite partie pénétrante (1) est constituée par une tige comprenant une première électrode extérieure (2). Ladite électrode (2) est disposée latéralement et sur toute la périphérie de ladite partie pénétrante (1).

Une seconde électrode (3), disposée dans la partie pénétrante (1) dudit instrument de pénétration, s'étend longitudinalement dans celle-ci et est isolée de la première électrode (2) par un isolant (4).

Lesdites électrodes (2, 3) et ledit isolant (4) sont, dans cet exemple de réalisation, avantageusement coaxiaux. Il est entendu que la portée de l'invention n'est en aucune manière limitée à une telle disposition desdites électrodes (2, 3).

Comme illustré par les figures 2 et 3, la partie pénétrante (1) dudit instrument de pénétration présente une partie longitudinale amovible, dont le retrait permet la réalisation de gestes chirurgicaux succédant l'acte de perçage de la structure osseuse.

Avantageusement, la partie longitudinale retirée de la partie pénétrante (1) dudit instrument de pénétration peut être replacée dans celle-ci, de sorte que ledit instrument de pénétration peut être réutilisé dans sa fonction première, à savoir le perçage de la structure osseuse.

Selon une configuration qui n'est pas l'invention, la partie longitudinale amovible constitue une partie interne de la partie pénétrante (1) dudit instrument de pénétration. Avantageusement, ladite partie longitudinale amovible est constituée par ladite électrode (3) interne (figure 2).

Ainsi, lorsque l'électrode (3) interne est retirée de la partie pénétrante (1) dudit instrument de pénétration, un canal opérateur est formé dans ledit instrument de pénétration.

De par la disposition desdites électrodes (2, 3) dans la partie pénétrante (1) dudit instrument de pénétration, ledit canal opérateur (5) est central.

Ledit canal (5) permet alors d'effectuer différentes opérations telles que le passage d'un embout d'injection d'un fluide (par exemple du ciment osseux), l'insertion d'une fibre optique ou d'un capteur d'image en vue par exemple d'une aide au diagnostic, ou encore le passage d'une broche de guidage pour le placement d'une vis cannelée pour la fixation osseuse. Dans ce dernier cas, une fois la broche de guidage glissée dans le canal opérateur (5), la partie pénétrante (1) dudit instrument de pénétration est retirée de la cavité formée dans la structure osseuse, laissant dans la cavité la seule broche de guidage. La vis cannelée est alors placée dans la structure osseuse au moyen de la broche de guidage.

Dans cet exemple de réalisation, la partie amovible est constituée de la seule électrode (3) interne. Il est entendu que, selon une autre configuration la partie longitudinale amovible peut comporter, outre ladite électrode (3) interne, ledit isolant (4).

Selon l'invention, la partie longitudinale amovible constitue la périphérie latérale de la partie pénétrante (1) dudit instrument de pénétration.

Avantageusement, ladite partie longitudinale amovible est constituée de ladite électrode (2) externe et dudit isolant (4) (figure 3).

Ainsi, lorsque la partie longitudinale amovible est retirée, seule reste dans la cavité (7) formée dans la structure osseuse ladite électrode (3) laquelle forme une âme de guidage (6).

Cette âme de guidage (6) constituée de ladite électrode (3) sert de guidage, par exemple, pour placer une vis cannelée.

De même que précédemment, de par la disposition desdites électrodes (2, 3) dans ledit instrument de pénétration (1), l'âme de guidage (6) est disposée centralement dans la cavité osseuse.

Dans cet exemple de réalisation, ladite âme de guidage est constituée de la seule électrode (3) interne. Il est entendu que, selon une autre configuration de l'invention, la partie longitudinale amovible peut ne concerner que la seule électrode (2) externe. Dans ces conditions, l'âme de guidage sera constituée de l'isolant (4) et de ladite électrode (3).

L'invention est décrite dans ce qui précède à titre d'exemple. Il est entendu que l'homme du métier est à même de réaliser différentes variantes de l'invention sans pour autant sortir du cadre du brevet.

## Revendications

1. Dispositif pour le suivi de la pénétration d'un instrument (1) dans une structure anatomique, en particulier une structure osseuse, comportant une source de tension alimentant au moins deux électrodes (2, 3), une desdites électrodes au moins étant portée par la partie (1) dudit instrument de pénétration destinée à pénétrer dans la structure anatomique, ainsi qu'un moyen de mesure de l'impédance entre lesdites électrodes (2, 3), dans lequel la partie pénétrante (1) dudit instrument de pénétration présente au moins une partie longitudinale amovible,
le dispositif étant **caractérisé en ce que** la partie longitudinale amovible constitue la périphérie latérale de la partie pénétrante dudit instrument de sorte que, lorsque la partie longitudinale est retirée, ladite partie pénétrante (1) dudit instrument de pénétration présente une âme de guidage (6).

2. Dispositif selon la revendication 1, **caractérisé en ce que** la partie pénétrante (1) dudit instrument de pénétration comporte en outre un canal opérateur (5) comprenant la partie longitudinale amovible.

3. Dispositif selon la revendication 2, **caractérisé en ce que** le canal opérateur (5) comprend une électrode amovible (3).

4. Dispositif selon la revendication 2 ou la revendication 3, **caractérisé en ce que** ledit dispositif comporte en outre une broche de guidage, souple ou rigide, traversant ledit canal opérateur (5).

5. Dispositif selon la revendication 2 ou selon la revendication 3, **caractérisé en ce que** ledit dispositif comporte en outre un tube d'injection de fluide traversant ledit canal opérateur (5).

6. Dispositif selon l'une quelconque des revendications 1 à 5, **caractérisé en ce que** l'âme de guidage (6) consiste en une électrode.

7. Dispositif selon l'une quelconque des revendications 1 à 6, **caractérisé en ce que** l'âme de guidage (6) est centrale.

8. Dispositif selon l'une quelconque des revendications 1 à 7, **caractérisé en ce que** la partie longitudinale amovible est une électrode (2, 3).

9. Dispositif selon l'une quelconque des revendications 1 à 8, comprenant en outre un électrostimulateur.

## Patentansprüche

1. Vorrichtung zum Verfolgen der Penetration eines Instruments in eine anatomische Struktur, insbesondere eine Knochenstruktur, umfassend eine Spannungsquelle, die mindestens zwei Elektroden (2, 3) versorgt, wobei zumindest eine der Elektroden durch den Teil (1) des Penetrationsinstruments getragen wird, das vorgesehen ist zum Penetrieren in die anatomische Struktur, sowie ein Mittel zum Messen der Impeanz zwischen den Elektroden (2, 3), worin der penetrierende Teil (1) des Penetrationsinstruments mindestens einen abnehmbaren longitudinalen Teil aufweist,
wobei die Vorrichtung **dadurch gekennzeichnet ist, dass** der abnehmbare longitudinale Teil die seitliche Peripherie des penetrierenden Teils des Instruments bildet, sodass, wenn der longitudinale Teil zurückgezogen wird, der penetrierende Teil (1) des Penetrationsinstruments als Führungsseele dient.

2. Vorrichtung nach Anspruch 1, **dadurch gekennzeichnet, dass** der penetrierende Teil (1) des Penetrationsinstruments weiterhin einen Betriebskanal (5) aufweist, der den abnehmbaren longitudinalen Teil umfasst.

3. Vorrichtung nach Anspruch 2, **dadurch gekennzeichnet, dass** der Betriebskanal (5) eine abnehmbare Elektrode (3) umfasst.

4. Vorrichtung nach Anspruch 2 oder Anspruch 3, **dadurch gekennzeichnet, dass** die Vorrichtung weiterhin einen Führungsdorn aufweist, flexibel oder steif, der durch den Betriebskanal läuft.

5. Vorrichtung nach Anspruch 2 oder Anspruch 3, **dadurch gekennzeichnet, dass** die Vorrichtung weiterhin eine Injektionsröhre ausweist, die durch den Betriebskanal läuft.

6. Vorrichtung nach einem der Ansprüche 1 bis 5, **dadurch gekennzeichnet, dass** die Führungsseele (6) aus einer Elektrode besteht.

7. Vorrichtung nach einem der Ansprüche 1 bis 6, **dadurch gekennzeichnet, dass** die Führungsseele (6) zentral ist.

8. Vorrichtung nach einem der Ansprüche 1 bis 7, **dadurch gekennzeichnet, dass** der abnehmbare longitudinale Teil eine Elektrode (2, 3) ist.

9. Vorrichtung nach einem der Ansprüche 1 bis 8, weiterhin umfassend einen Elektrostimulator.

## Claims

1. Device to monitor penetration of an instrument (1) into an anatomical structure, in particular a bone structure, comprising a voltage source supplying at least two electrodes (2, 3), at least one of said electrodes being carried by the part (1) of said penetration instrument intended to penetrate the anatomical structure, as well as means to measure the impedance between said electrodes (2, 3), wherein the penetrating part (1) of said penetration instrument presents at least one removable longitudinal part,
the device being **characterized in that** the removable longitudinal part forms a lateral periphery of the penetrating part of said instrument so that, when the longitudinal part is removed, said penetrating part (1) of said penetration instrument presents a guide core (6).

2. Device according to claim 1, **characterized in that** the penetrating part (1) of said penetration instrument further comprises an operator channel (5) comprising the removable longitudinal part.

3. Device according to claim 12, **characterized in that** the operator channel (5) comprises a removable electrode (3).

4. Device according to claim 2 or claim 3, **characterized in that** said device further comprises a flexible or rigid guide pin, crossing the operator channel (5).

5. Device according to claim 2 or claim 3, **characterized in that** said device further comprises a fluid injection tube crossing the operator channel (5).

6. Device according to any of claims 1 to 5, **characterized in that** the guide core (6) consists in an electrode.

7. Device according to any of claims 1 to 6, **characterized in that** the guide core (6) is central.

8. Device according to any of claims 1 to 7, **characterized in that** the removable longitudinal part is an electrode (2, 3).

9. Device according to any of claims 1 to 8, further comprising an electro-stimulator.
